# EUROPEAN PATENT APPLICATION

(11) **EP 2 309 198 A1**
(43) Date of publication of application: **13.04.2011**
(21) Application number: 09769880.7
(22) Date of filing: 22.06.2009
(51) Int. Cl.: F24F 7/10, A61L 9/00, A61L 9/22, B03C 3/02, B03C 3/40, B03C 3/47, F24F 7/00

(54) **VENTILATION DEVICE**

(30) Priority: 24.06.2008 JP 2008164097
(71) Applicant: Daikin Industries, Ltd., Osaka-shi, Osaka 530-8323 (JP)
(72) Inventor: KAGAWA, Kenkichi, Sakai-shi Osaka 591-8511 (JP); TANAKA, Toshio, Sakai-shi Osaka 591-8511 (JP); ODA, Yasuhiro, Kusatsu-shi Shiga 525-8526 (JP)
(74) Representative: Goddar, Heinz J.
(86) International application number: PCT/JP2009/002830
(87) International publication number: WO 2009/157170

(57) **Abstract**

A ventilator (10) includes a casing (20) which includes an inlet (21) for outdoor air, an inlet (22) for indoor air, and an air outlet (23), and an air-blowing fan (30) contained in the casing (20). The ventilator (10) is configured to suck the outdoor air and the indoor air through the corresponding inlets (21, 22) into the casing (20) by using the air-blowing fan (30), process the sucked air, and supply the processed air through the outlet (23) into a room. An electrical dust collecting mechanism (40) is provided in the casing (20) to remove dust in the sucked air. Furthermore, a deodorizing mechanism (50) having a low resistance to air circulation is provided in the casing (20) to remove an odorous substance in the sucked air.

## Description

### TECHNICAL FIELD

The present invention relates to ventilators, and more particularly relates to a measure for reducing the pressure loss of circulating air.

### BACKGROUND ART

Conventionally, some ventilators have been placed outdoors as described in PATENT DOCUMENT 1. Such a ventilator is placed on the ceiling of a balcony of an apartment, and includes an outdoor air processor and an indoor air processor. The outdoor air processor includes a dust collecting mechanism, a deodorizing mechanism, and an air-blowing fan which are contained in a casing.

The ventilator is configured such that while indoor air is taken out by the indoor air processor, the outdoor air processor takes in the indoor air taken out by the indoor air processor, and outdoor air. The outdoor air processor collects dust from the taken-in air by using the dust collecting mechanism, and then, deodorizes the taken-in air by using the deodorizing mechanism, thereby supplying the resultant air into a room.

### CITATION LIST

### PATENT DOCUMENT

PATENT DOCUMENT 1: Japanese Patent No. 3908179

### SUMMARY OF THE INVENTION

### TECHNICAL PROBLEM

However, in a conventional ventilator, a photocatalyst body made of small beads is used as a deodorizing mechanism, and thus, a problem occurs in which the resistance to the air circulation is high, and the pressure loss is high. As a result, a high-capacity air-blowing fan needs to be used, thereby increasing the size of the entire ventilator.

The present invention has been made in view of the foregoing point, and it is an object of the present invention to provide a ventilator with a low resistance to the air circulation.

### SOLUTION TO THE PROBLEM

A first aspect of the invention is directed to a ventilator including a casing (20) which includes an inlet (21) for outdoor air, an inlet (22) for indoor air, and an air outlet (23), and an air-blowing fan (30) contained in the casing (20), and configured to suck the outdoor air and the indoor air through the corresponding inlets (21, 22) into the casing (20) by using the air-blowing fan (30), process the sucked air, and supply the processed air through the outlet (23) into a room. An electrical dust collecting mechanism (40) is provided in the casing (20) to remove dust in the sucked air. Furthermore, a deodorizing mechanism (50) having a low resistance to air circulation is provided in the casing (20) to remove an odorous substance in the sucked air.

In the first aspect of the invention, when the air-blowing fan (30) is driven, outdoor air and indoor air are sucked through the corresponding inlets (21, 22) into the casing (20). In the casing (20), the electrical dust collecting mechanism (40) removes dust in the sucked air. Furthermore, the deodorizing mechanism (50) having a low resistance to the air circulation removes odorous substances in the sucked air. The air processed by the dust collecting mechanism (40) and the deodorizing mechanism (50) is supplied through the outlet (23) into the room.

A second aspect of the invention is the ventilator according to the first aspect of the invention, wherein the dust collecting mechanism (40) includes an air passage (66, 76) extending along an air flow in the casing (20) from both the inlets (21, 22) to the outlet (23).

In the second aspect of the invention, the air passage (66, 76) of the dust collecting mechanism (40) extends along the air flow. This allows the resistance to the air circulation to be low, thereby providing a low pressure loss.

A third aspect of the invention is the ventilator according to the first or second aspect of the invention, wherein the deodorizing mechanism (50) includes an air passage (56) extending along an air flow in the casing (20) from both the inlets (21, 22) to the outlet (23).

In the third aspect of the invention, the air passage (56) of the deodorizing mechanism (50) extends along the air flow. This allows the resistance to the air circulation to be low, thereby providing a low pressure loss.

A fourth aspect of the invention is the ventilator according to the second aspect of the invention, wherein the dust collecting mechanism (40) includes a charging portion (42), and a dust collecting portion (43) for collecting dust electrically charged by the charging portion (42). The dust collecting portion (43) includes a first electrode (60) and a second electrode (70) each including a grid-like base (61, 71) having a large number of air passages (66, 76) extending along the air flow, and a large number of projections (62, 72) extending from the base (61, 71) in a direction parallel to an axial direction of the air passages (66, 76), the projections (62) of the first electrode (60) each extend toward an inside of the corresponding air passage (76) of the second electrode (70), and the projections (72) of the second electrode (70) each extend toward an inside of the corresponding air passage (66) of the first electrode (60).

In the fourth aspect of the invention, the dust charged by the charging portion (42) is adsorbed on, e.g., the first electrode (60), and is collected. Since, in particular, the dust collecting portion (43) has a large number of air passages (66, 76) extending along the air flow, this allows the resistance to the air circulation to be low, thereby providing a low pressure loss.

A fifth aspect of the invention is the ventilator according to the third aspect of the invention, wherein the deodorizing mechanism (50) includes an electrical discharge section (51) for generating plasma in the sucked air, and a catalyst filter (52) placed downstream of the electrical discharge section (51) to accelerate decomposition of an odorous substance in the sucked air. The catalyst filter (52) includes a large number of air passages (56) extending along the air flow.

In the fifth aspect of the invention, the plasma generated by the electrical discharge section (51) contains a highly reactive substance (active species, such as electrons, ions, ozone, or radicals), and thus, the highly reactive substance decomposes and removes odorous substances in the sucked air. Thereafter, when the highly reactive substance reaches the catalyst filter (52), the highly reactive substance is further activated, thereby decomposing and removing odorous substances in the sucked air. Since, in particular, the catalyst filter (52) has a large number of air passages (56) extending along the air flow, this allows the resistance to the air circulation to be low, thereby providing a low pressure loss.

A sixth aspect of the invention is the ventilator according to any one of the first through fifth aspects of the invention, wherein the casing (20) is placed indoors.

In the sixth aspect of the invention, the casing (20) is placed indoors, thereby enabling indoor dust collection and indoor deodorization.

### ADVANTAGES OF THE INVENTION

According to the present invention, the electrical dust collecting mechanism (40) and the deodorizing mechanism (50) with a low resistance to the air circulation are disposed in the casing (20). Therefore, the resistance to the air circulation is low, resulting in a significant reduction in the pressure loss. This enables the use of the small-capacity air-blowing fan (30), thereby downsizing the entire apparatus.

According to the second aspect of the invention, the dust collecting mechanism (40) is an electrical mechanism, and includes the air passage (66, 76) extending along the air flow in the casing (20) from the inlets (21, 22) to the air outlet (23). This can reduce the resistance to the air circulation in the entire apparatus, thereby reducing the pressure loss.

According to the third aspect of the invention, the deodorizing mechanism (50) includes the air passage (56) extending along the air flow in the casing (20) from the inlets (21, 22) to the air outlet (23). This can reduce the resistance to the air circulation in the entire apparatus, thereby reducing the pressure loss.

According to the fourth aspect of the invention, the dust collecting portion (43) is formed in a grid structure. Therefore, while the dust collecting portion (43) can increase the area in which dust is collected, the dust collecting portion (43) can include the air passages (66, 76) extending along the air flow in the casing (20) from the inlets (21, 22) to the air outlet (23). This can reduce the resistance to the air circulation in the entire apparatus, thereby reducing the pressure loss.

According to the fifth aspect of the invention, the electrical discharge section (51) of the deodorizing mechanism (50) includes two electrodes, and the catalyst filter (52) of the deodorizing mechanism (50) is formed in a monolith structure. Therefore, the deodorizing mechanism (50) includes the air passages (56) extending along the air flow in the casing (20) from the inlets (21, 22) to the air outlet (23). As a result, the deodorizing mechanism (50) can reduce the resistance to the air circulation. Therefore, the entire structure of the deodorizing mechanism (50) has low pressure loss, thereby downsizing the entire apparatus.

When the casing (20) is placed indoors, and is configured as a so-called ceiling-mounted casing, a duct through which outdoor air, etc., is taken and a duct through which processed air is supplied indoors need to be connected to the casing (20), thereby increasing the resistance to the air circulation through the ducts. According to the sixth aspect of the invention, the resistance to the air circulation in the casing (20) is reduced, thereby reducing the pressure loss in the entire system including the ducts.

### BRIEF DESCRIPTION OF THE DRAWINGS

[FIG. 1] FIG. 1 is a cross-sectional side view illustrating the entire structure of a ventilator of a first embodiment of the present invention.
[FIG. 2] FIG. 2 is a cross-sectional top view of the ventilator of the first embodiment.
[FIG. 3] FIG. 3 is a perspective view illustrating a dust collecting unit of the first embodiment.
[FIG. 4] FIG. 4 is a front view illustrating a charging portion of the dust collecting unit of the first embodiment.
[FIG. 5] FIG. 5 is a side view illustrating the charging portion of the dust collecting unit of the first embodiment with a side plate omitted.
[FIG. 6] FIG. 6 is a perspective view illustrating a dust collecting portion of the first embodiment.
[FIG. 7] FIG. 7 is an enlarged perspective view illustrating a portion of the dust collecting portion of the first embodiment.
[FIG. 8] FIG. 8 is an enlarged cross-sectional side view illustrating a portion of the dust collecting portion of the first embodiment.
[FIG. 9] FIG. 9 is a perspective view illustrating a catalyst filter of a deodorizing unit of the first embodiment.
[FIG. 10] FIG. 10 is a front view illustrating a charging portion of a dust collecting unit of a second embodiment of the present invention.
[FIG. 11] FIG. 11 is a side view illustrating the charging portion of the dust collecting unit of the second embodiment with a side plate omitted.
[FIG. 12] FIG. 12 is an enlarged cross-sectional side view illustrating a portion of a dust collecting portion of a third embodiment of the present invention.

### DESCRIPTION OF EMBODIMENTS

Embodiments of the present invention will be described hereinafter in detail with reference to the drawings.

### <First Embodiment of the Invention>

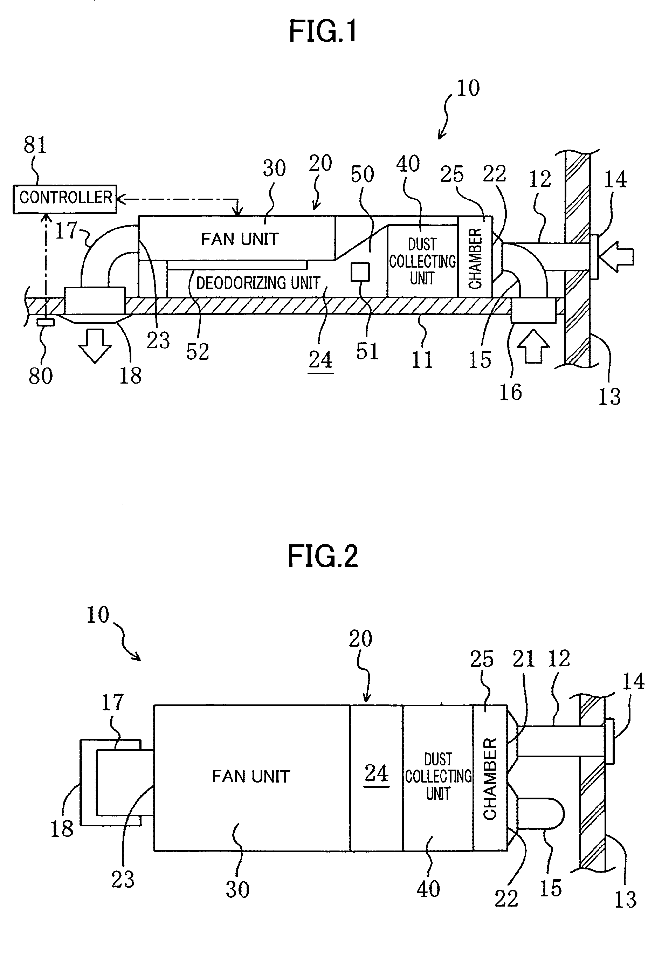

As illustrated in FIGS. 1-9, a ventilator (10) of a first embodiment is placed indoors.

As illustrated in FIGS. 1 and 2, the ventilator (10) includes a casing (20), an air-blowing fan (30), a dust collecting unit (40), and a deodorizing unit (50).

The casing (20) is formed as a flat rectangular body including a rectangular top plate, a rectangular bottom plate, front and rear side plates extending widthwise, and right and left side plates extending lengthwise. The casing (20) is placed on the back face (top face) of a ceiling (11) of a building.

An inlet (21) through which outdoor air is sucked, and an inlet (22) through which indoor air is sucked are formed in the front side plate of the casing (20) so as to be aligned laterally, and an outlet (23) for air is formed in an upper part of a central portion of the rear side plate.

One end of a duct (12) is connected to the inlet (21) for the outdoor air, the duct (12) passes through a wall (13) of the building, and the other end of the duct (12) is extended outdoors, and includes a suction unit (14), such as a grille, attached to the wall (13). One end of a duct (15) is connected to the inlet (22) for the indoor air, the duct (15) is bent downward, and the other end of the duct (15) is extended to the ceiling (11), and includes a suction unit (16), such as a grille, attached to the ceiling (11). One end of a duct (17) is connected to the air outlet (23), the duct (17) is bent downward, and the other end of the duct (17) is extended to the ceiling (11), and includes a discharge unit (18), such as a diffuser, attached to the ceiling (11).

The interior of the casing (20) is configured as a circulation passage (24) through which air flows from both of the inlets (21, 22) to the outlet (23). The circulation passage (24) in the interior of the casing (20) includes a chamber (25), the dust collecting unit (40), the deodorizing unit (50), and the air-blowing fan (30) which are placed along the air flow from the front of the inlets (21, 22) to the back of the outlet (23).

The chamber (25) is formed inside the front side plate. The chamber (25) is formed in which the outdoor air taken through the inlet (21) for outdoor air and the indoor air taken through the inlet (22) for indoor air are collected while being mixed.

The air-blowing fan (30) forms a fan unit, and is disposed in an upper back portion of the interior of the casing (20). Although not shown, the air-blowing fan (30) includes a single fan, or multiple fans.

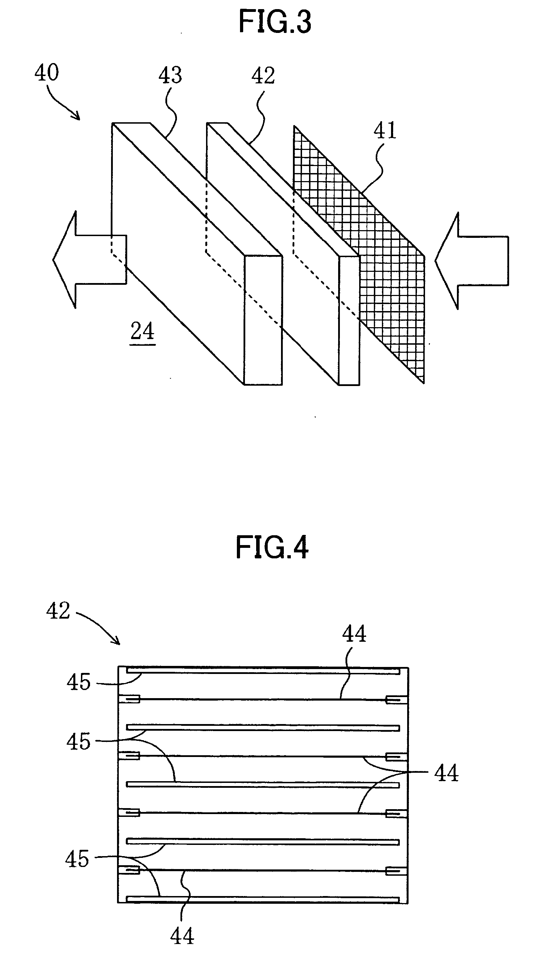

The dust collecting unit (40) is placed downstream of the chamber (25) while being adjacent to the chamber (25). As illustrated in FIG. 3, the dust collecting unit (40) includes a prefilter (41), a charging portion (42), and a dust collecting portion (43) which are arranged in this order from the upstream to the downstream side of air flow, and forms an electrical dust collecting mechanism.

The prefilter (41) forms a filter for collecting relatively large dust contained in sucked air which is a mixture of the outdoor air and indoor air taken in by the chamber (25).

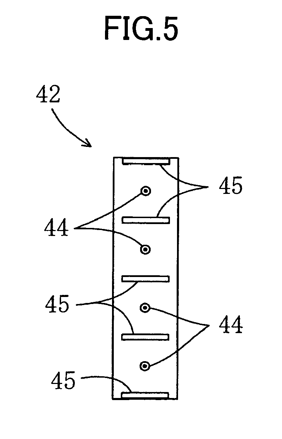

The charging portion (42) forms an ionizer to charge relatively small dust passing through the prefilter (41). As illustrated in FIGS. 4 and 5, the charging portion (42) includes a plurality of discharge electrodes (44) and a plurality of opposed electrodes (45), and is configured such that a direct-current voltage is applied between each of the discharge electrodes (44) and the corresponding opposed electrode (45) to charge dust in the sucked air.

The discharge electrodes (44) are formed by ionizing wires, are disposed to extend from the left end of the charging portion (42) to the right end thereof, and are arranged vertically in parallel. The opposed electrodes (45) are disposed to extend from the left end of the charging portion (42) to the right end thereof, and are arranged vertically in parallel. The discharge electrodes (44) are disposed, one between each adjacent pair of the opposed electrodes (45). Therefore, a space located in the charging portion (42) and between each discharge electrode (44) and an adjacent opposed electrode (45) is formed as an air passage extending along the air flow in the casing (20) from the inlets (21, 22) to the outlet (23). Specifically, a space between the discharge electrode (44) and the adjacent opposed electrode (45) forms an air passage parallel to the circulation passage (24) in the casing (20), and has a low resistance to the air circulation.

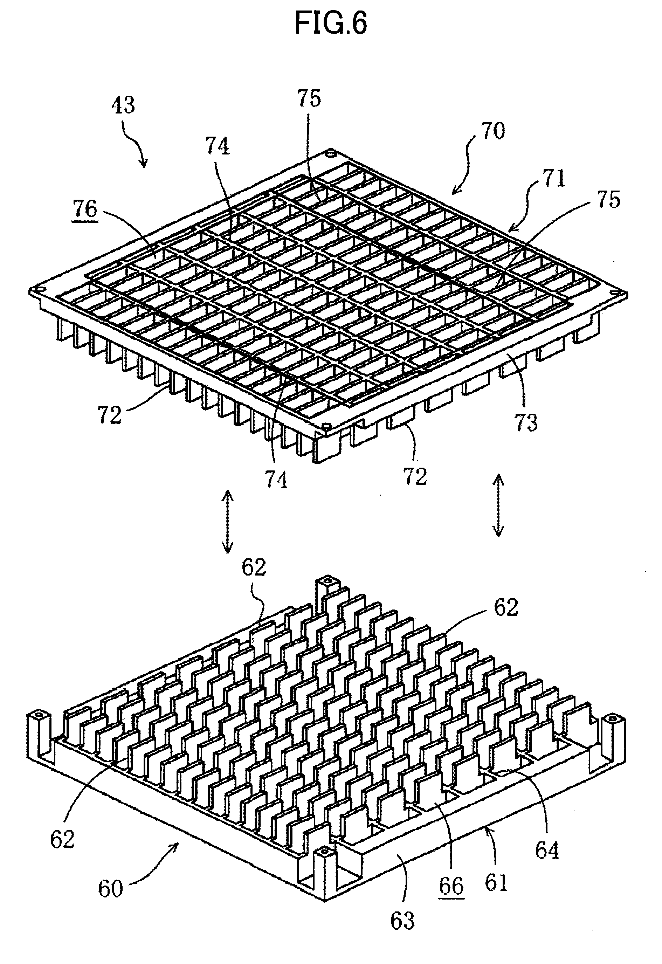

The dust collecting portion (43) is configured to collect dust charged in the charging portion (42) by adsorption, and includes, as illustrated in FIGS. 6-8, a dust collecting electrode (60) which is an earth electrode, and a high-voltage electrode (70) which is a positive electrode. Any one of the dust collecting electrode (60) and the high-voltage electrode (70) forms a first electrode, and the other one thereof forms a second electrode.

The dust collecting electrode (60) and the high-voltage electrode (70) are both made of a conductive resin, and are each formed, as a single-piece structure, by integral molding. The dust collecting electrode (60) and the high-voltage electrode (70) are formed in substantially the same shape, and configured in an insertion structure in which they can be partly inserted into each other.

The dust collecting electrode (60) and the high-voltage electrode (70) are both preferably made of a slightly conductive resin, and furthermore, the volume resistivity of the resin is preferably greater than or equal to 10⁸ Ωcm and less than 10¹³ Ωcm.

Each of the dust collecting electrode (60) and the high-voltage electrode (70) is formed in a rectangular shape, and includes a single base (61, 71), and a large number of projections (62, 72) projecting from the base (61, 71). The base (61, 71) includes a frame (63, 73), a plurality of vertical partitions (64, 74) arranged within the frame (63, 73), and a plurality of horizontal partitions (65, 75) arranged within the frame (63, 73).

The frame (63, 73) is formed in a rectangular shape. The frame (63) of the dust collecting electrode (60) and the frame (73) of the high-voltage electrode (70) are fixed to each other at their four corners, and thus, the base (61) of the dust collecting electrode (60) and the base (71) of the high-voltage electrode (70) are disposed to face each other. Furthermore, the bases (61, 71) of the dust collecting electrode (60) and the high-voltage electrode (70) are oriented in a direction orthogonal to the air flow in the circulation passage (24).

The vertical partitions (64, 74) of the dust collecting electrode (60) and the high-voltage electrode (70) extend vertically, while the horizontal partitions (65, 75) thereof extend in a width direction. The vertical partitions (64, 74) and the horizontal partitions (65, 75) are arranged to crisscross each other. Each base (61, 71) has a large number of air passages (66, 76) formed therein and surrounded by the frame (63, 73), the vertical partitions (64, 74), and the horizontal partitions (65, 75). In other words, the base (61, 71) is formed in a rectangular grid structure by the vertical partitions (64, 74) and the horizontal partitions (65, 75), thereby forming a large number of tubular portions to form the air passages (66, 76).

The air passages (66, 76) are formed as passages extending along the air flow in the casing (20) from the inlets (21, 22) to the outlet (23). Specifically, the air passages (66, 76) form passages parallel to the circulation passage (24) in the casing (20), and has a low resistance to the air circulation.

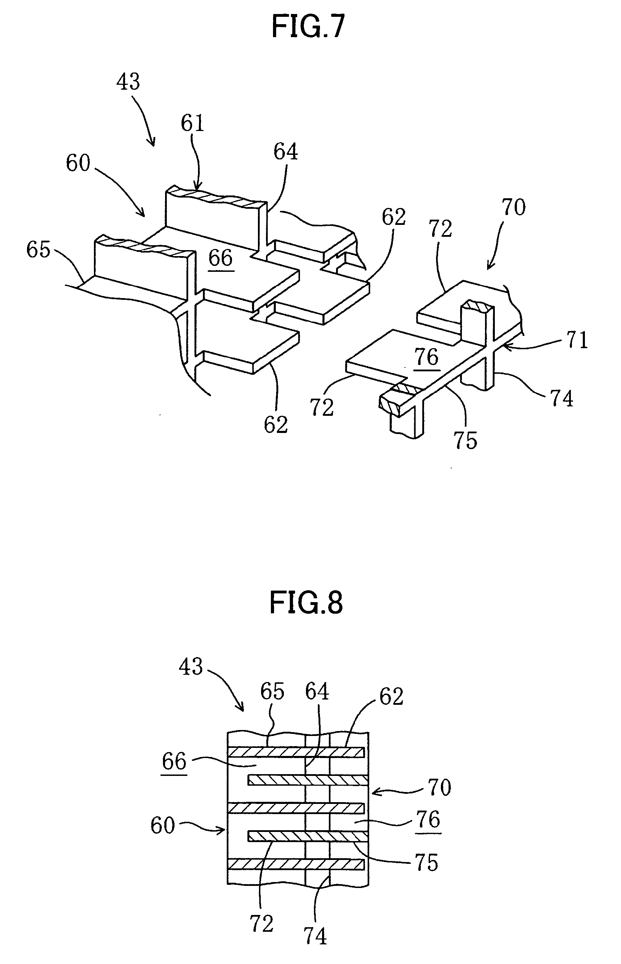

Each of the vertical partitions (64) of the dust collecting electrode (60) and a corresponding one of the vertical partitions (74) of the high-voltage electrode (70) are formed to be in the same plane in an assembled state where the base (61) of the dust collecting electrode (60) and the base (71) of the high-voltage electrode (70) are locked with each other. On the other hand, the horizontal partitions (65) of the dust collecting electrode (60) and the horizontal partitions (75) of the high-voltage electrode (70) are formed to be alternately arranged in a vertically staggered pattern in FIGS. 7 and 8 in the assembled state where the base (61) of the dust collecting electrode (60) and the base (71) of the high-voltage electrode (70) are locked with each other. In other words, the horizontal partitions (65) of the dust collecting electrode (60) are located in the middle of the air passages (76) of the high-voltage electrode (70), while the horizontal partitions (75) of the high-voltage electrode (70) are located in the middle of the air passages (66) of the dust collecting electrode (60).

The projections (62, 72) are integrally connected to the corresponding horizontal partitions (65, 75) to project from the corresponding horizontal partitions (65, 75). The projections (62, 72) are formed into projecting pieces in the shape of a flat plate having the same thickness as the horizontal partitions (65, 75), and extend toward the inside of the corresponding air passages (66, 76) of the opposed electrodes (60, 70). Furthermore, the projections (62, 72) are formed so that each of the vertical partitions (74, 64) of the opposed electrodes (70, 60) is located in a clearance between each horizontally adjacent pair of the projections (62, 72).

The projections (62, 72) are each located in the middle of the corresponding air passage (76, 66) in the assembled state where the base (61) of the dust collecting electrode (60) and the base (71) of the high-voltage electrode (70) are locked with each other, and thus, air flows above and below the projections (62, 72). Each projection (62) of the dust collecting electrode (60) and the adjacent projection (72) of the high-voltage electrode (70) are configured to have a distance of 1.0-2.0 mm therebetween. For example, the distance between the projection (62) and the adjacent projection (72) is preferably 1.2 mm.

The vertical partitions (64) of the dust collecting electrode (60) and the vertical partitions (74) of the high-voltage electrode (70) are located a predetermined distance apart from and without contact with each other in the assembled state where the base (61) of the dust collecting electrode (60) and the base (71) of the high-voltage electrode (70) are locked with each other.

Specifically, each projection (62) of the dust collecting electrode (60) is surrounded by the corresponding vertical partitions (74) and horizontal partitions (75) of the high-voltage electrode (70), and has equal distances from the surrounding vertical partitions (74) and horizontal partitions (75), thereby creating a radial electric field in the cross section of the corresponding air passage (76). Furthermore, each projection (72) of the high-voltage electrode (70) is surrounded by the corresponding vertical partitions (64) and horizontal partitions (65) of the dust collecting electrode (60), and has equal distances from the surrounding vertical partitions (64) and horizontal partitions (65), thereby creating a radial electric filed in the cross section of the corresponding air passage (66).

A direct-current voltage is applied between the dust collecting electrode (60) and the high-voltage electrode (70) to create an electric field therebetween, and thus, electrically charged dust is adsorbed on the dust collecting electrode (60).

The deodorizing unit (50) is disposed downstream of the dust collecting unit (40) to extend from the dust collecting unit (40) to the air-blowing fan (30). Specifically, the deodorizing unit (50) is provided to extend from an outlet in the back face of the dust collecting unit (40) to an inlet in the bottom face of the air-blowing fan (30). The deodorizing unit (50) is configured as a deodorizing mechanism with a low resistance to the air circulation such that an electrical discharge section (51) and a catalyst filter (52) are arranged in this order from the upstream to the downstream side of air flow.

Although not shown, the electrical discharge section (51) includes discharge electrodes and opposed electrodes, and the discharge electrodes are disposed, one between each adjacent pair of the opposed electrodes. The opposed electrodes are formed by, e.g., linear or rod-like electrodes, and are arranged in substantially parallel with the discharge electrodes. Specifically, a space located in the electrical discharge section (51) and between each discharge electrode and an adjacent opposed electrode (45) is formed as an air passage extending along the air flow in the casing (20) from the inlets (21, 22) to the outlet (23). Specifically, a space between the discharge electrode and the adjacent opposed electrode forms an air passage parallel to the circulation passage (24) in the casing (20), and has a low resistance to the air circulation.

The electrical discharge section (51) is configured to generate a streamer discharge. When the electrical discharge section (51) generates a streamer discharge, low-temperature plasma is generated. The low-temperature plasma contains a highly reactive substance (active species, such as electrons, ions, ozone, or radicals), and thus, the highly reactive substance in the low-temperature plasma decomposes and removes odorous substances including toxic substances in the air.

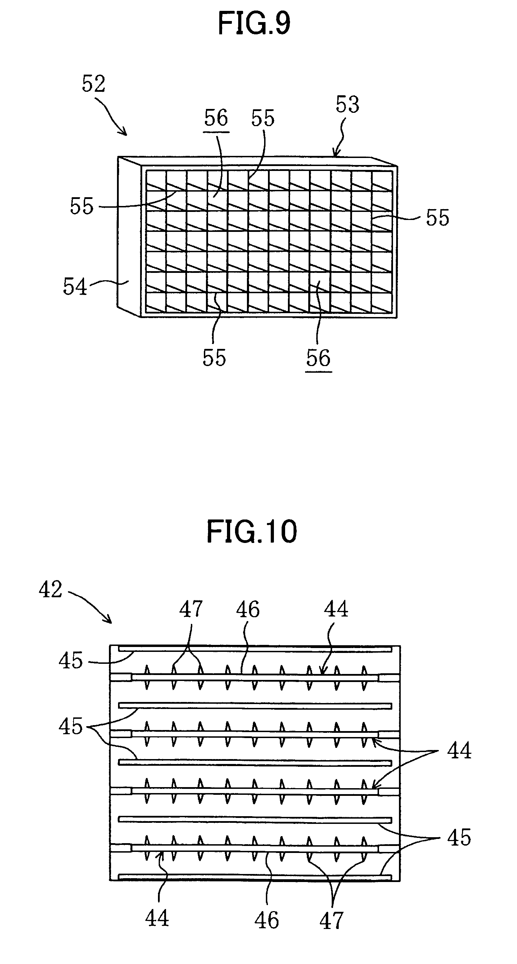

The catalyst filter (52) is disposed downstream of the electrical discharge section (51) while being adjacent to the inlet in the bottom face of the air-blowing fan (30). As illustrated in FIG. 9, the catalyst filter (52) is configured to carry a catalyst on the surface of a base material (53). The base material (53) has a monolith structure in which a large number of partitions (55) are formed vertically and horizontally inside a rectangular frame material (54), and a large number of air passages (56) are formed inside the frame material (54) by the partitions (55). The air passages (56) are formed as passages extending along the air flow in the casing (20) from the inlets (21, 22) to the outlet (23). Specifically, the air passages (56) form passages parallel to the circulation passage (24) in the casing (20), and each have a low resistance to the air circulation.

Manganese catalysts, precious metal catalysts, etc., are used as the catalyst, and such a catalyst further activates a highly reactive substance in low-temperature plasma generated by discharge, and accelerates the decomposition and removal of odorous substances in the sucked air.

By contrast, a pressure sensor (80) is placed indoors. The indoor pressure is sensed by the pressure sensor (80), and the sensed pressure is transmitted to a controller (81). The controller (81) controls the air volume of the air-blowing fan (30) in order to allow the indoor pressure to be positive.

### -Operational Behavior-

Next, the ventilation operation of the above-described ventilator (10) will be described.

First, when the air-blowing fan (30) is driven, outdoor air is sucked through the inlet (21) into the casing (20), and indoor air is sucked through the inlet (22) into the casing (20). The outdoor air and indoor air sucked into the casing (20) collect in the chamber (25), and are mixed, and the sucked air which is a mixture of the sucked indoor and outdoor air flows into the dust collecting unit (40).

In the dust collecting unit (40), while a direct-current voltage is applied between each discharge electrode (44) of the charging portion (42) and the corresponding opposed electrode (45) thereof, a direct-current voltage is applied between the dust collecting electrode (60) and the high-voltage electrode (70) of the dust collecting portion (43).

The sucked air passes through the prefilter (41), thereby collecting relatively large dust contained in the sucked air. The sucked air having passed through the prefilter (41) flows into the charging portion (42). In the charging portion (42), relatively small dust having passed through the prefilter (41) is charged with electricity to take a positive charge, for example, and the electrically charged dust flows downstream.

The electrically charged dust flows into the dust collecting portion (43), and flows through the air passages (66, 76) in the bases (61, 71) of the dust collecting electrode (60) and the high-voltage electrode (70). Specifically, the indoor air flows through the air passages (66, 76) formed by the frames (63, 73), the vertical partitions (64, 74), and the horizontal partitions (65, 75) of the bases (61, 71) of the dust collecting electrode (60) and the high-voltage electrode (70), and flows around each of the projections (62, 72) of the dust collecting electrode (60) and the high-voltage electrode (70).

Since, in this case, the dust collecting electrode (60) serves as, e.g., an earth electrode, and is set to a negative electrode, the dust charged with positive electricity is adsorbed on the dust collecting electrode (60). Specifically, the dust is adsorbed on the inner surface of the frame (63) of the dust collecting electrode (60), the surfaces of the vertical partitions (64) thereof, the surfaces of the horizontal partitions (65) thereof, and the surfaces of the projections (62) thereof.

By contrast, in the deodorizing unit (50), the electrical discharge section (51) generates a streamer discharge, thereby generating low-temperature plasma. The sucked air flowing through the dust collecting unit (40) flows into the deodorizing unit (50), and passes through the streamer discharge. The low-temperature plasma contains a highly reactive substance (active species, such as electrons, ions, ozone, or radicals), and thus, the highly reactive substance removes odorous substances including toxic substances in the sucked air. Thereafter, the sucked air flows into the catalyst filter (52). When the highly reactive substance reaches the catalyst filter (52), the highly reactive substance is further activated, thereby decomposing and removing odorous substances in the sucked air.

Clean processed air from which the dust is removed and from which toxic substances and odorous substances are also removed flows through the air-blowing fan (30), and is blown through the outlet (23) into the room.

### -Advantages of First Embodiment-

As described above, according to the first embodiment, the electrical dust collecting unit (40) and the deodorizing unit (50) with a low resistance to the air circulation are disposed in the casing (20). Therefore, the resistance to the air circulation is low, resulting in a significant reduction in the pressure loss. This enables the use of the small-capacity air-blowing fan (30), thereby downsizing the entire apparatus.

A duct through which outdoor air, etc., is taken and a duct through which processed air is supplied indoors need to be connected, in particular, to the ventilator (10) having the casing (20) placed in the indoor ceiling, i.e., the so-called ceiling-mounted ventilator (10), thereby increasing the resistance to the air circulation through the ducts. Therefore, the resistance to the air circulation in the casing (20) is reduced, thereby reducing the pressure loss in the entire system including the ducts.

Furthermore, the dust collecting unit (40) is an electrical unit, and the charging portion (42) and the dust collecting portion (43) each include electrodes of two types. Therefore, the dust collecting unit (40) includes an air passage extending along the air flow in the casing (20) from the inlets (21, 22) to the air outlet (23). As a result, the dust collecting unit (40) can reduce the resistance to the air circulation, thereby reducing the pressure loss.

In particular, the dust collecting portion (43) is formed in a grid structure. Therefore, while the dust collecting portion (43) can increase the area in which dust is collected, the dust collecting portion (43) can include the air passages (66, 76) extending along the air flow in the casing (20) from the inlets (21, 22) to the air outlet (23). As a result, the dust collecting unit (40) can reduce the resistance to the air circulation, thereby reducing the pressure loss.

The electrical discharge section (51) of the deodorizing unit (50) includes two electrodes, and the catalyst filter (52) of the deodorizing unit (50) is formed in a monolith structure. Therefore, the deodorizing unit (50) includes the air passage (56) extending along the air flow in the casing (20) from the inlets (21, 22) to the air outlet (23). As a result, the deodorizing unit (50) can reduce the resistance to the air circulation. Therefore, the entire structure of the deodorizing unit (50) has low pressure loss, thereby downsizing the entire apparatus.

Since outdoor air and indoor air are both taken in, heat can be more advantageously utilized than when only outdoor air is taken in.

Since the casing (20) is placed indoors, only a single opening can be formed in a wall of a building to take in outdoor air.

### <Second Embodiment of the Invention>

Next, a second embodiment of the present invention will be described in detail with reference to the drawings.

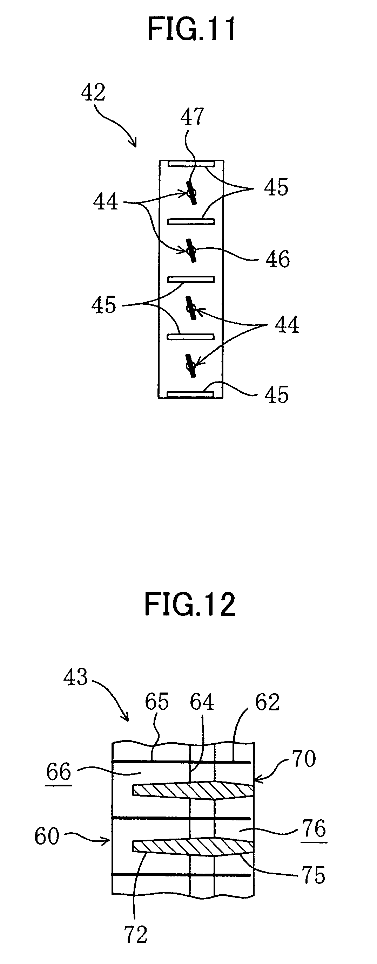

In the second embodiment, as illustrated in FIGS. 10 and 11, the discharge electrodes (44) of the charging portion (42) of the dust collecting unit (40) are formed by serrated electrodes instead of by ionizing wires in the first embodiment.

Specifically, the discharge electrodes (44) each include an electrode rod (46), and needles (47) formed on the electrode rod (46). The electrode rod (46) is disposed to extend from the left end of the charging portion (42) to the right end thereof. The plurality of needles (47) are formed to project toward the opposed electrodes (45). In other words, the needles (47) project upward and downward. Therefore, an electrical current is discharged from the needles (47) to the opposed electrodes (45). The other structures, operations, and advantages are similar to those of the first embodiment.

### <Third Embodiment of the Invention>

Next, a third embodiment of the present invention will be described in detail with reference to the drawings.

In the first embodiment, the dust collecting electrode (60) and the high-voltage electrode (70) of the dust collecting portion (43) of the dust collecting unit (40) are both made of a conductive resin. However, in the third embodiment, as illustrated in FIG. 12, the dust collecting electrode (60) is made of a conductive metal.

Specifically, the dust collecting electrode (60) is made of a metal sheet, such as stainless steel, while the high-voltage electrode (70) is made of a conductive resin similarly to the first embodiment.

Similarly to the first embodiment, the dust collecting electrode (60) is formed in a rectangular shape, and includes a single base (61) and a large number of projections (62), and the base (61) includes a frame (63), a plurality of vertical partitions (64), and a plurality of horizontal partitions (65). The projections (62), the frame (63), the vertical partitions (64), and the horizontal partitions (65) are each made of a metal sheet of a conductive metal.

Similarly to the first embodiment, the projections (62) of the dust collecting electrode (60) extend toward the inside of the air passage (76) of the high-voltage electrode (70). Meanwhile, similarly to the first embodiment, the projections (72) of the high-voltage electrode (70) extend toward the inside of the air passage (66) of the dust collecting electrode (60).

Therefore, according to this embodiment, the dust collecting electrode (60) is made of a conductive metal. This allows the metal sheet to be thinner than the resin, thereby improving the efficiency of integration and downsizing the entire apparatus. The other structures, operations, and advantages are similar to those of the first embodiment.

In this embodiment, the dust collecting electrode (60) is made of a conductive metal, and the high-voltage electrode (70) is made of a conductive resin. However, the dust collecting electrode (60) may be made of a conductive resin, and the high-voltage electrode (70) may be made of a conductive metal.

### <Other Embodiments>

The first embodiment of the present invention may be configured as follows.

In the above embodiments, the catalyst filter (52) of the deodorizing unit (50) is formed in a monolith structure. However, the catalyst filter (52) may have a honeycomb structure or a corrugated structure. In short, the catalyst filter (52) may be configured to include an air passage extending along the air flow in the casing (20) from the inlets (21, 22) to the air outlet (23).

The deodorizing unit (50) includes the electrical discharge section (51) and the catalyst filter (52). However, the deodorizing unit (50) may include only the electrical discharge section (51). Specifically, the electrical discharge section (51) may be configured to also decompose odorous substances. The deodorizing unit (50) may include only the catalyst filter (52). Specifically, the catalyst filter (52) may include activated carbon, etc., and the catalyst filter (52) may be configured to also decompose odorous substances.

The electrical discharge process of the electrical discharge section (51) of the deodorizing unit (50) is not limited to a streamer discharge. Various electrical discharge processes, such as a pulse discharge, may be used as the discharge process.

The foregoing embodiments have been set forth merely for purposes of preferred examples in nature, and are not intended to limit the scope, applications, and use of the invention.

### INDUSTRIAL APPLICABILITY

As described above, the present invention is useful for a ventilator for collecting dust and removing odors.

### DESCRIPTION OF REFERENCE CHARACTERS

- 10: Ventilator
- 20: Casing
- 30: Air-Blowing Fan
- 40: Dust Collecting Unit (Dust Collecting Mechanism)
- 42: Charging Portion
- 43: Dust Collecting Portion
- 50: Deodorizing Unit (Deodorizing Mechanism)
- 51: Electrical Discharge Section
- 52: Catalyst Filter
- 56: Air Passage
- 60: Dust Collecting Electrode
- 70: High-Voltage Electrode
- 61, 71: Base
- 62, 72: Projection
- 66, 76: Air Passage

## Claims

1. A ventilator including a casing (20) which includes an inlet (21) for outdoor air, an inlet (22) for indoor air, and an air outlet (23), and an air-blowing fan (30) contained in the casing (20), and configured to suck the outdoor air and the indoor air through the corresponding inlets (21, 22) into the casing (20) by using the air-blowing fan (30), process the sucked air, and supply the processed air through the outlet (23) into a room, the ventilator comprising:
an electrical dust collecting mechanism (40) provided in the casing (20) to remove dust in the sucked air; and
a deodorizing mechanism (50) provided in the casing (20) to remove an odorous substance in the sucked air, and having a low resistance to air circulation.

2. The ventilator of claim 1, wherein
the dust collecting mechanism (40) includes an air passage (66, 76) extending along an air flow in the casing (20) from both the inlets (21, 22) to the outlet (23).

3. The ventilator of claim 1 or 2, wherein
the deodorizing mechanism (50) includes an air passage (56) extending along an air flow in the casing (20) from both the inlets (21, 22) to the outlet (23).

4. The ventilator of claim 2, wherein
the dust collecting mechanism (40) includes a charging portion (42), and a dust collecting portion (43) for collecting dust electrically charged by the charging portion (42),
the dust collecting portion (43) includes a first electrode (60) and a second electrode (70) each including a grid-like base (61, 71) having a large number of air passages (66, 76) extending along the air flow, and a large number of projections (62, 72) extending from the base (61, 71) in a direction parallel to an axial direction of the air passages (66, 76), the projections (62) of the first electrode (60) each extend toward an inside of the corresponding air passage (76) of the second electrode (70), and the projections (72) of the second electrode (70) each extend toward an inside of the corresponding air passage (66) of the first electrode (60).

5. The ventilator of claim 3, wherein
the deodorizing mechanism (50) includes an electrical discharge section (51) for generating plasma in the sucked air, and a catalyst filter (52) placed downstream of the electrical discharge section (51) to accelerate decomposition of an odorous substance in the sucked air, and
the catalyst filter (52) includes a large number of air passages (56) extending along the air flow.

6. The ventilator of claim 1, wherein
the casing (20) is placed indoors.
